# EUROPÄISCHE PATENTSCHRIFT

(11) **EP 1 625 876 B1**
(45) Veröffentlichungstag und Bekanntmachung des Hinweises auf die Patenterteilung: **13.05.2009**
(21) Anmeldenummer: 05107254.4
(22) Anmeldetag: 05.08.2005
(51) Int. Cl.: A61N 5/10

(54) **Vorrichtung zur Durchführung einer Bestrahlungstherapie**
Device for radiotherapy
Dispositif pour radiotherapie

(30) Priorität: 12.08.2004 DE 102004039191
(43) Veröffentlichungstag der Anmeldung: 15.02.2006
(73) Patentinhaber: SIEMENS AKTIENGESELLSCHAFT, 80333 München (DE)
(72) Erfinder: Sommer, Andres, 91094 Langensendelbach (DE)

(56) Entgegenhaltungen:
- EP-A- 1 380 262
- WO-A-01/60236
- DE-U1- 20 109 313
- US-A1- 2002 085 668
- US-A1- 2002 188 194
- US-B1- 6 516 046

## Beschreibung

Die Erfindung betrifft eine Vorrichtung zur Ermittlung und Überwachung von Parametern einer Bestrahlungstherapie. Eine Strahlentherapie-Einrichtung ist beispielsweise aus der DE 199 04 675 A1 bekannt.

Vor Durchführung einer Strahlentherapie werden die zu bestrahlenden Körperregionen typischerweise mit einem Bild gebenden medizintechnischen Verfahren, insbesondere Computertomographie-Verfahren, untersucht. Die Aufnahmen, die vom in der zu untersuchenden Körperregion immobilisierten Patienten erstellt werden, werden als Datensatz gespeichert. Mit Hilfe dieses Datensatzes können Parametereinstellungen einer Bestrahlungseinrichtung, insbesondere betreffend Felder oder Bestrahlungsarten, vorgenommen werden. Ebenso ist es möglich, vor der Programmierung der Bestrahlungseinrichtung die Bestrahlung zu simulieren. Bei Durchführung der Bestrahlung wird davon ausgegangen, dass sich Geometrie und sonstige Eigenschaften des zu bestrahlenden Gewebes im Vergleich zur Situation bei der Diagnose mittels des Bild gebenden medizintechnischen Verfahrens nicht geändert haben. Sofern sich im Laufe der Strahlentherapie Eigenschaften des zu bestrahlenden Gewebes ändern, hat dies zur Folge, dass die gewählte Parametereinstellung der Bestrahlungseinrichtung nur noch in geringerem Maße auf die Erfordernisse der Bestrahlung abgestimmt ist.

Aus der DE 102 10 050 A1 geht ein Verfahren zur Repositionierung eines Patienten in einer diagnostisch/therapeutischen Anlage und eine solche Anlage hervor. Hierbei werden bei einer ersten Sitzung Referenzaufnahmen von dem Patienten oder einem Teil des Patienten mittels zwei Videokameras erstellt. Diese werden bei einer nachfolgenden Sitzung mit den aktuellen Aufnahmen verglichen und die Position des Patienten wird so lange verändert, bis die sichtbaren Positionsunterschiede minimiert sind.

Aus der WO 01/60236 ist eine Vorrichtung zur Durchführung einer Bestrahlungstherapie zu entnehmen, die neben einer Bestrahlungseinrichtung zum Erzeugen von Röntgen- oder Partikelstrahlen ein bildgebendes Diagnosegerät in Form eines Cone-Beam-Computertomographen umfasst. Dabei ist der Cone-Beam-Computertomograph derart ausgestaltet, direkt während einer Bestrahlungsbehandlung die Lage des zu bestrahlenden Gewebes zu erfassen und die Bestrahlungseinrichtung entsprechend anzupassen. Es handelt sich insgesamt um eine so genannte On-line-Behandlungsplanung, wobei die On-line gewonnenen 3D-Bildaufnahmen des Cone-Beam-Computertomographen direkt zu einer veränderten Bestrahlung führen.

Der Erfindung liegt die Aufgabe zugrunde, eine einfache und zuverlässige Überwachung einer Strahlentherapie zu ermöglichen.

Vor Durchführung der Bestrahlungsbehandlung wird mittels der Vorrichtung nach einem der Ansprüche 1 bis 5 eine erste Aufnahme des zu bestrahlenden Gewebes mittels eines Bild gebenden medizintechnischen Verfahrens, insbesondere eines Computertomographie-Verfahrens erstellt. Mit Hilfe dieser ersten Aufnahme erfolgt eine erste Parametereinstellung einer Bestrahlungseinrichtung, beispielsweise eines LINAC oder einer Partikelbestrahlungsmaschine. Die Parametereinstellung kann dabei unter Nutzung von Bilddaten manuell, teilautomatisch oder vollautomatisch erfolgen. Mit der damit festgelegten Parametereinstellung wird nun das zu behandelnde Gewebe bestrahlt. Nach einer oder mehreren Bestrahlungen wird mindestens eine weitere Aufnahme des zu bestrahlenden Gewebes mittels des Bild gebenden medizintechnischen Verfahrens erstellt. Die zu verschiedenen Zeitpunkten erstellten Aufnahmen werden automatisch verglichen, wobei ein so genannter Entscheider im Fall einer einen Schwellwert übersteigenden Abweichung der Aufnahmen automatisch ein Abweichungssignal generiert. Dieses Abweichungssignal weist den Bediener der Bestrahlungseinrichtung darauf hin, dass sich die Eigenschaften des zu bestrahlenden Gewebes derart geändert haben, dass die Parametereinstellung zu ändern ist. Vorzugsweise ist der mindestens eine Schwellwert, der festlegt, ab wann ein Abweichungssignal erzeugt wird, einstellbar. Damit ist es dem Bediener möglich, die Empfindlichkeit der Überwachung von Veränderungen des zu bestrahlenden Gewebes, verknüpft mit der Überwachung der Parametereinstellung der Bestrahlungseinrichtung, je nach betroffener Körperregion unterschiedlich einzustellen.

Der Vergleich der der Parametereinstellung zugrunde liegenden Aufnahmen bezieht sich vorzugsweise sowohl auf das Zielgebiet der Bestrahlung, d.h. in der Regel auf den Tumor, als auch auf das in Einstrahlrichtung außerhalb des Zielgebietes befindliche Gewebe. Bevorzugt werden vom Zielgebiet und vom zu bestrahlenden Gewebe außerhalb des Zielgebiets sowohl geometrische Eigenschaften als auch sonstige Eigenschaften, insbesondere Schwächungs- oder Dichtewerte, automatisch ausgewertet. Relevante geometrische Eigenschaften sind insbesondere Lage und Form des Tumors sowie Volumen und durchstrahlte Weglänge in Einstrahlrichtung zwischen Haut und Zielgebiet. Vorzugsweise sind mehrere Schwellwerte, beispielsweise betreffend geometrische Werte einerseits und Dichtewerte andererseits, unabhängig einstellbar.

Nach einer vorteilhaften Weiterbildung zeigt das mittels eines Vergleichers und Entscheiders generierte Abweichungssignal nicht nur an, dass die Parametereinstellung der Bestrahlungseinrichtung geändert werden sollte, sondern generiert zugleich einen Planungshinweis für die Parametereinstellung. Dies trägt erheblich zur Vereinfachung der Bedienung der Bestrahlungseinrichtung bei, wobei gleichzeitig die Gefahr von Fehleinstellungen von Parametern der Bestrahlungseinrichtung minimiert wird. Hierbei kann die mittels fest vorgegebener oder einstellbarer Algorithmen empfohlene neue Parametereinstellung einer automatischen Plausibilitätskontrolle unterzogen werden. Eine nochmalige Rationalisierung der Bedienung ist nach einer besonders vorteilhaften Weiterbildung dadurch gegeben, dass die Parametereinstellung der Bestrahlungseinrichtung bei einer über dem Schwellwert liegenden Abweichung zwischen zu verschiedenen Zeitpunkten erstellten Aufnahmen des zu bestrahlenden Gewebes automatisch den geänderten Bedingungen angepasst wird. Hierbei kann zweckmäßigerweise vorgesehen sein, dass die neue Parametereinstellung nur nach Freigabe durch den Benutzer wirksam wird. Damit ist eine zusätzliche Kontrolle der automatisch vorgeschlagenen Parameteränderung gegeben.

Der Vorteil der Erfindung liegt insbesondere darin, dass im Verlauf einer Bestrahlungsbehandlung erstellte Aufnahmen, insbesondere Computertomographie-Aufnahmen, nicht nur genutzt werden, um Änderungen des bestrahlten Gewebes festzustellen, sondern zugleich herangezogen werden, um auf besonders rationelle, vorzugsweise zumindest teilweise automatisierte Art Betriebsparameter der Bestrahlungseinrichtung in Anpassung an die geänderte Gewebestruktur zu ändern.

Nachfolgend wird ein Ausführungsbeispiel der Erfindung anhand einer Zeichnung näher erläutert. Hierin zeigen in schematischer Darstellung:
- FIG 1: ein Verfahren zur Überwachung von Parametern einer Bestrahlungstherapie, und
- FIG 2: eine Vorrichtung zur Durchführung einer Bestrahlungstherapie.

Einander entsprechende Teile oder Parameter sind in beiden Figuren mit den gleichen Bezugszeichen gekennzeichnet.

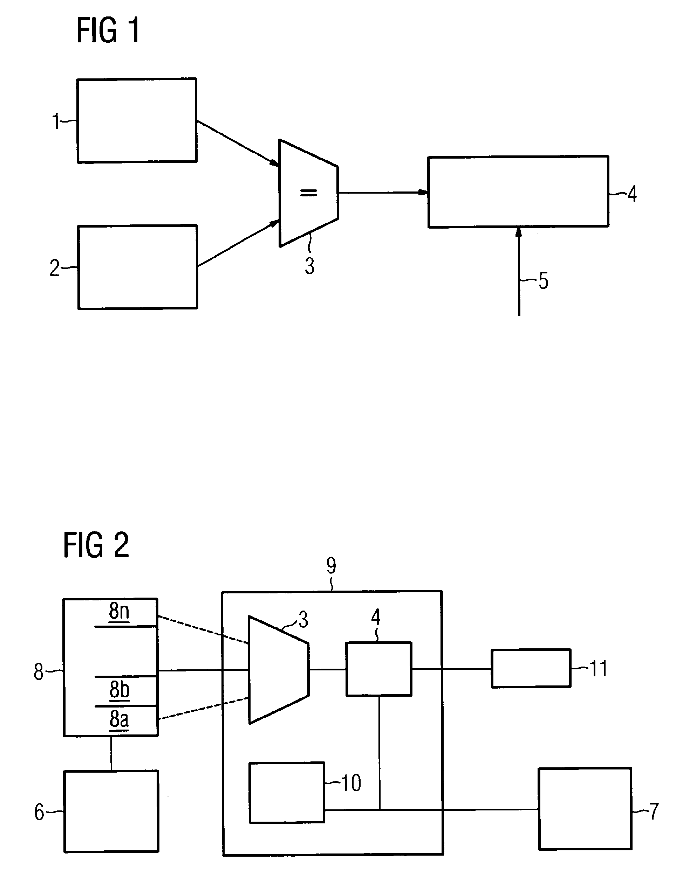

Im Schema nach FIG 1 wird davon ausgegangen, dass als Planungsgrundlage einer Bestrahlungstherapie mehrerer Aufnahmen des zu bestrahlenden Zielgebiets existieren. Die Aufnahmen sind mittels eines Bild gebenden medizintechnischen Verfahrens, insbesondere Computertomographie- oder Magnetresonanz-Verfahren, erstellt und als Planungsbilder 1 und aktuelle Bilder 2 bezeichnet. Die bei jeweils einer Untersuchung gewonnenen Bilder werden, im Fall einer Computertomographie-Untersuchung, auch als CT-Datensatz bezeichnet. Die Planungsbilder 1 wurden vor Beginn der Bestrahlungsbehandlung erstellt und wurden zur Einstellung eines Parametersatzes einer Bestrahlungseinrichtung, beispielsweise Photonen- oder Partikelbestrahlungsmaschine, verwendet. Bei der Einstellung der Parameter der Bestrahlungseinrichtung werden geometrische und sonstige, insbesondere Strahlen beeinflussende Eigenschaften sowohl des zu bestrahlenden Zielgewebes, d.h. des Tumors, als auch des in Einstrahlrichtung ebenfalls der Strahlung ausgesetzten Gewebes berücksichtigt.

Um festzustellen, ob die Parameter der Bestrahlungseinrichtung noch geeignet eingestellt sind, wird ein automatischer Vergleich der Planungsbilder 1 mit den aktuellen Bildern 2, die vor oder nach einer oder mehreren Bestrahlungen erstellt wurden, durchgeführt. Die Bestrahlung des Patienten erfolgt beispielsweise täglich. Ebenso werden beispielsweise täglich oder in größeren Zeitabständen Computertomographie-Aufnahmen des zu behandelnden Gewebes erstellt. Im Fall einer Protonenstrahltherapie wird mit Hilfe der Computertomographie vor jeder Bestrahlung die Position korrigiert. Die hierbei gewonnenen Daten können gleichzeitig für die Abweichungskontrolle anderer Parameter der Bestrahlung genutzt werden.

Der Überwachung der Parametereinstellung der Bestrahlungseinrichtung, beispielsweise einer Ionen-Bestrahlungsmaschine oder einer elektromagnetische Strahlung aussendenden Einrichtung, dient ein Vergleicher 3, der auf die gespeicherten Planungsbilder 1 sowie auf die aktuellen Bilder 2 zugreift. In den Vergleich werden geometrische Merkmale, d.h. insbesondere Lage und Form des Tumors sowie des diesen umgebenden Gewebes, sowie sonstige Merkmale, insbesondere die Strahlenabsorption von Gewebe, einbezogen. In Abhängigkeit vom Grad der Abweichung zwischen dem mindestens einen aktuellen Bild 2 und dem mindestens einen Planungsbild 1 stellt ein Entscheider 4 fest, ob die Parametereinstellung der Bestrahlungseinrichtung noch geeignet ist oder geändert werden sollte. Die Schwelle, ab welcher eine Änderung der Parametereinstellung empfohlen wird, ist durch mindestens einen Schwellwert 5 einstellbar. Bei einer Schwellwertüberschreitung wird automatisch ein Abweichungssignal, beispielsweise ein durch eine Datenverarbeitungsanlage verarbeitbares Signal, welches aus mehreren Einzelsignalen in beliebiger Formatierung gebildet sein kann, generiert. Gegebenenfalls aus dem Abweichungssignal erzeugte, insbesondere durch den Benutzer visuell wahrnehmbare Signale, welche auf eine aufgrund Veränderungen des bestrahlten Gewebes vorzunehmende Änderung der Parametereinstellung der Bestrahlungseinrichtung hinweisen, werden ebenfalls unter dem Begriff Abweichungssignal subsumiert.

Bei besonders empfindlichem Gewebe in unmittelbarer Nähe des zu bestrahlenden Tumors kann der Schwellwert 5 bzw. können die Schwellwerte 5 beispielsweise besonders niedrig eingestellt werden. Ergibt sich aus dem automatischen Vergleich der aktuellen Bilder 2 mit den Planungsbildern 1 eine Schwellwertüberschreitung, so werden die detektierten Veränderungen beispielsweise durch eine farbliche Markierung auf einer der Darstellungen 1,2, insbesondere auf dem aktuellen Bild 2, veranschaulicht. Eine solche Visualisierung der Veränderung des bestrahlten Gewebes erleichtert die Festlegung geänderter Parameter der Bestrahlungseinrichtung. Diese Parameter legen insbesondere die Bestrahlungsgeometrie und die Dosisverteilung im Tumor und seiner Umgebung sowie die zeitliche Verteilung der für die Bestrahlungsbehandlung erforderlichen Gesamtdosis fest. Vorzugsweise werden geänderte Parameter dem Bediener durch das beschriebene System automatisch vorgeschlagen. Die Tätigkeit des Bedieners bei der Neueinstellung der Parameter der Bestrahlungseinrichtung, beispielsweise der Einstellung von Feldern und Bestrahlungsarten, kann sich darauf beschränken, die geänderten Parameter zu kontrollieren und freizugeben.

Die in FIG 2 symbolisiert dargestellte Vorrichtung zur Durchführung einer Bestrahlungstherapie weist eine das vorstehend beschriebene Verfahren ermöglichende Verknüpfung zwischen einem Bild gebenden Diagnosegerät 6, nämlich einem Computertomographiegerät, und einer Bestrahlungseinrichtung 7 zur Tumorbehandlung auf. Die mittels des Diagnosegerätes 6 erstellten Aufnahmen werden in einem Bildspeicher 8, der verschiedene Speicherbereiche 8a,8b,.... 8n aufweist, gespeichert. Dabei ist beispielsweise das Planungsbild 1 im Speicherbereich 8a und das aktuelle Bild 2 im Speicherbereich 8n abgelegt. Die zu verschiedenen Zeitpunkten aufgenommenen Bilder 1,2 werden, wie durch gestrichelte Linien angedeutet, an den Vergleicher 3 übermittelt. Der Vergleicher 3 ist Teil eines Planungsrechners 9, der in der symbolisierten Darstellung als geschlossene Einheit dargestellt ist, jedoch ebenso aus mehreren miteinander verknüpften Einzelgeräten gebildet sein kann. Ebenso kann abweichend von der Darstellung nach FIG 2 der Bildspeicher 8 ein Teil des Planungsrechners 9 oder verschiedene der im Folgenden beschriebenen Komponenten des Planungsrechners 9 zu einheitlichen Bauteilen zusammengefasst sein. Insbesondere ist es möglich, den Vergleicher 3 sowie den Entscheider 4 softwaretechnisch zu realisieren. In jedem Fall dient der Planungsrechner 9 vor Beginn der Bestrahlungsbehandlung dazu, auf Grundlage der ersten Aufnahmen, das heißt der Planungsbilder 1, eine erste Parametereinstellung der Bestrahlungseinrichtung 7 zu ermitteln.

Der an den Vergleicher 3 angeschlossene Entscheider 4 weist eine Verbindung mit einem Parameterspeicher 10 auf, in welchem die Parameter zum Betrieb einer Bestrahlungseinrichtung 7, die der Tumorbehandlung dient, gespeichert sind. Im Parameterspeicher 10 können verschiedene Parametereinstellungen gespeichert werden, die jeweils auf zu bestimmten Zeitpunkten aufgenommene Merkmale des zu bestrahlenden Gewebes abgestimmt sind. Durch eine Zuordnung der zu verschiedenen Zeitpunkten aufgenommenen, in den Speicherbereichen 8a .... 8n abgelegten Bilder 1,2 der betroffenen Gewebebereiche des Patienten zu den verschiedenen im Parameterspeicher 10 gespeicherten Parametereinstellungen der Bestrahlungseinrichtung 7 sind wesentliche Daten des Verlaufs der Bestrahlungstherapie übersichtlich dokumentiert. Die Therapie wird auch als bildgeführte Strahlentherapie oder adaptive Strahlentherapie (ART) bezeichnet.

Die Empfindlichkeit des Entscheiders 4 kann bei Bedarf nach jeder neuen Aufnahme 1,2 neu eingestellt werden. Die entsprechenden Schwellwerte 5 werden mittels einer Eingabevorrichtung 11, die Teil des Planungsrechners 9 oder an diesen angeschlossen ist, eingegeben. Ebenso ist es möglich, dass der Planungsrechner 9 in Abhängigkeit von Merkmalen des der Bestrahlung ausgesetzten Gewebes die Schwellwerte 5 dem Benutzer vorschlägt oder automatisch einstellt. In jedem Fall sichert der automatische Vergleich der aktuellen Bilder 2 mit den Planungsbildern 1 eine hohe Qualität der Bestrahlung über die gesamte Dauer der Bestrahlungstherapie.

## Patentansprüche

1. Vorrichtung zur Durchführung einer Bestrahlungstherapie, mit
- einem Bild gebenden Diagnosegerät (6),
- einem Bildspeicher (8) zur Speicherung von mit dem Diagnosegerät (6) gewonnenen Aufnahmen (1,2),
- einer mit einer wählbaren Parametereinstellung betreibbaren Bestrahlungseinrichtung (7),
- einem zur Ermittlung und Überwachung der Parametereinstellung vorgesehenen Planungsrechner (9), welcher
- einen Vergleicher (3) zum Vergleich verschiedener mit dem Diagnosegerät (6) gewonnener Aufnahmen (1,2),
- einen Entscheider (4) zur Auswertung des mit dem Vergleicher (3) durchgeführten Vergleichs, und
- einen Parameterspeicher (10) zur Speicherung der Parametereinstellung aufweist,
**dadurch gekennzeichnet,**
**dass** der Entscheider ausgebildet ist zum Generieren eines Abweichungssignals im Fall einer einen Schwellwert (5) übersteigenden Abweichung der Aufnahmen (1,2).

2. Vorrichtung nach Anspruch 1,
**dadurch gekennzeichnet, dass** das Bild gebende Diagnosegerät (6) ein Computertomograph ist.

3. Vorrichtung nach Anspruch 1 oder 2,
**dadurch gekennzeichnet, dass** die Bestrahlungseinrichtung (7) eine Partikelstrahlungsmaschine umfasst.

4. Vorrichtung nach einem der Ansprüche 1 bis 3,
**gekennzeichnet durch** eine zur Einstellung des Schwellwerts (5) des Entscheiders (4) vorgesehene Eingabevorrichtung (11).

5. Vorrichtung nach einem der Ansprüche 1 bis 4,
**dadurch gekennzeichnet, dass** sich der Vergleich der Aufnahmen (1,2) auf Eigenschaften des zu bestrahlenden Gewebes bezieht.

6. Vorrichtung nach einem der Ansprüche 1 bis 5,
**dadurch gekennzeichnet, dass** sich der Vergleich der Aufnahmen (1,2) auf Eigenschaften des Gewebes in Einstrahlrichtung außerhalb des zu bestrahlenden Gewebes bezieht.

7. Vorrichtung nach einem der Ansprüche 1 bis 6,
**dadurch gekennzeichnet, dass** sich der Vergleich der Aufnahmen (1,2) auf geometrische Eigenschaften des Gewebes bezieht.

8. Vorrichtung nach einem der Ansprüche 1 bis 7,
**dadurch gekennzeichnet, dass** sich der Vergleich der Aufnahmen (1,2) auf Strahlen beeinflussende Eigenschaften des Gewebes bezieht.

9. Vorrichtung nach einem der Ansprüche 1 bis 8,
**dadurch gekennzeichnet, dass** als Abweichungssignal ein Planungshinweis für die Parametereinstellung der Bestrahlungseinrichtung (7) generierbar ist.

10. Vorrichtung nach einem der Ansprüche 1 bis 9,
**dadurch gekennzeichnet , dass** mittels des Abweichungssignals die Parametereinstellung der Bestrahlungseinrichtung (7) automatisch änderbar ist.

11. Vorrichtung nach einem der Ansprüche 1 bis 10,
**dadurch gekennzeichnet, dass** mehrere, unabhängig voneinander einstellbare Schwellwerte vorgesehen sind.

## Claims

1. Device for performing radiotherapy, comprising
- an imaging diagnostic device (6),
- an image memory (8) for storing images (1,2) acquired by means of the diagnostic device (6),
- an irradiation apparatus (7) which can be operated by means of a selectable parameter setting,
- a planning computer (9) which is provided for determining and monitoring the parameter setting and which has
- a comparator (3) for comparing different images (1,2) acquired by means of the diagnostic device (6),
- a decider (4) for evaluating the comparison performed by the comparator (3), and
- a parameter memory (10) for storing the parameter setting,
**characterised in that**
the decider is embodied for generating a deviation signal if a difference between the images (1,2) exceeds a threshold value (5).

2. Device according to claim 1,
**characterised in that** the imaging diagnostic device (6) is a computed tomography device.

3. Device according to claim 1 or 2,
**characterised in that** the irradiation apparatus (7) comprises a particle radiation machine.

4. Device according to one of claims 1 to 3,
**characterised by** an input device (11) provided for setting the threshold value (5) of the decider (4).

5. Device according to one of claims 1 to 4,
**characterised in that** the comparison of the images (1,2) relates to properties of the tissue that is to be irradiated.

6. Device according to one of claims 1 to 5,
**characterised in that** the comparison of the images (1,2) relates to properties of the tissue in the radiation direction outside the tissue that is to be irradiated.

7. Device according to one of claims 1 to 6,
**characterised in that** the comparison of the images (1,2) relates to geometric properties of the tissue.

8. Device according to one of claims 1 to 7,
**characterised in that** the comparison of the images (1,2) relates to beam-influencing properties of the tissue.

9. Device according to one of claims 1 to 8,
**characterised in that** a planning instruction for performing the parameter setting of the irradiation apparatus (7) can be generated as a deviation signal.

10. Device according to one of claims 1 to 9,
**characterised in that** the parameter setting of the irradiation apparatus (7) can be adjusted automatically by means of the deviation signal.

11. Device according to one of claims 1 to 10,
**characterised in that** a plurality of threshold values which can be set independently of one another are provided.

## Revendications

1. Dispositif pour la mise en oeuvre d'une radiothérapie, avec
- un appareil de diagnostic (6) fournisseur d'images, une mémoire d'images (8) pour le stockage d'enregistrements (1, 2) obtenus avec l'appareil de diagnostic (6),
- une installation d'irradiation (7) exploitable avec sélection du réglage des paramètres,
- un ordinateur de planification (9) prévu pour la détermination et la surveillance du réglage des paramètres, qui présente
- un comparateur (3) pour la comparaison de différents enregistrements (1, 2) obtenus avec l'appareil de diagnostic (6),
- un décideur (4) pour l'évaluation de la comparaison réalisée avec le comparateur (3), et
- une mémoire des paramètres (10) pour le stockage du réglage des paramètres,
**caractérisé en ce que** le décideur est exécuté pour générer un signal d'écart dans le cas d'un écart des enregistrements (1, 2) dépassant une valeur limite (5).

2. Dispositif selon la revendication 1, **caractérisé en ce que** l'appareil de diagnostic fournisseur d'images (6) est un tomodensitomètre.

3. Dispositif selon la revendication 1 ou 2, **caractérisé en ce que** l'installation d'irradiation (7) comprend une machine de radiation de particules.

4. Dispositif selon l'une des revendications 1 à 3, **caractérisé par** un dispositif d'entrée (11) prévu pour le réglage de la valeur limite (5) du décideur (4).

5. Dispositif selon l'une des revendications 1 à 4, **caractérisé en ce que** la comparaison des enregistrements (1, 2) se rapporte à des propriétés du tissu à irradier.

6. Dispositif selon l'une des revendications 1 à 5, **caractérisé en ce que** la comparaison des enregistrements (1, 2) se rapporte à des propriétés du tissu dans la direction d'irradiation à l'extérieur du tissu à irradier.

7. Dispositif selon l'une des revendications 1 à 6, **caractérisé en ce que** la comparaison des enregistrements (1, 2) se rapporte à des propriétés géométriques du tissu à irradier.

8. Dispositif selon l'une des revendications 1 à 7, **caractérisé en ce que** la comparaison des enregistrements (1, 2) se rapporte à des propriétés du tissu influençant les rayons.

9. Dispositif selon l'une des revendications 1 à 8, **caractérisé en ce qu'**une indication de planification pour le réglage des paramètres de l'installation d'irradiation (7) peut être générée en tant que signal d'écart.

10. Dispositif selon l'une des revendications 1 à 9, **caractérisé en ce que** le réglage des paramètres de l'installation d'irradiation (7) peut être modifié automatiquement à l'aide du signal d'écart.

11. Dispositif selon l'une des revendications 1 à 10, **caractérisé en ce que** plusieurs valeurs limites réglables indépendamment sont prévues.
